Europäisches Patentamt

European Patent Office

Office européen des brevets

(19)

(11) Veröffentlichungsnummer: **0 039 413**
**B1**

(12) **EUROPÄISCHE PATENTSCHRIFT**

(45) Veröffentlichungstag der Patentschrift:
**16.03.83**

(21) Anmeldenummer: **81102523.8**

(22) Anmeldetag: **03.04.81**

(51) Int. Cl.³: **C 07 C 53/40**, C 07 C 51/58,
B 01 J 31/18

(54) Verfahren zur Herstellung von Acetylchlorid.

(30) Priorität: **02.05.80 DE 3016900**

(43) Veröffentlichungstag der Anmeldung:
**11.11.81 Patentblatt 81/45**

(45) Bekanntmachung des Hinweises auf die Patenterteilung:
**16.03.83 Patentblatt 83/11**

(84) Benannte Vertragsstaaten:
**AT BE CH DE FR GB IT LI NL**

(56) Entgegenhaltungen:
**DE-A-2 648 134**
**DE-C-897 554**

(73) Patentinhaber: **HOECHST AKTIENGESELLSCHAFT,**
**Postfach 80 03 20, D-6230 Frankfurt/Main 80 (DE)**

(72) Erfinder: **Erpenbach, Heinz, Dr., Oberbuschweg 22,**
**D-5000 Köln 50 (DE)**
Erfinder: **Gehrmann, Klaus, Dr.,**
**Geschwister-Scholl-Strasse 32, D-5042 Erftstadt (DE)**
Erfinder: **Lork, Winfried,**
**Siegfried-von-Westerburg-Strasse 14, D-5042 Erftstadt**
**(DE)**
Erfinder: **Prinz, Peter, Von-Geyr-Ring 104, D-5030 Hürth**
**(DE)**

## Verfahren zur Herstellung von Acetylchlorid

Die Erfindung betrifft ein Verfahren zur Herstellung von Acetylchlorid durch Umsetzung von Methylchlorid mit Kohlenmonoxid unter praktisch wasserfreien Bedingungen bei Temperaturen von 350 bis 575 K und Drücken von 1 bis 300 bar in Gegenwart eines Katalysatorsystems, welches mindestens eines der Edelmetalle Rhodium, Palladium, Iridium oder deren Verbindungen, Jod und/oder dessen Verbindungen, ggf. Trialkylphosphinoxid oder Triarylphosphinoxid, sowie ein inertes organisches Lösemittel enthält, welches dadurch gekennzeichnet ist, dass man in Gegenwart eines Katalysatorsystems arbeitet, welches n-Heptan als inertes Lösemittel und zusätzlich Methyltrialkylphosphoniumjodid und/oder Methyltriarylphosphoniumjodid und mindestens eine Verbindung des Chroms, Molybdäns oder Wolframs enthält.

Die japanische Offenlegungsschriften 53/46 912 (1978), 53/63 307 (1978) und 53/63 308 (1978) beschreiben bereits Verfahren zur Herstellung von Acetylhalogeniden aus Alkylhalogeniden und Kohlenmonoxid in Gegenwart eines Katalysatorsystems, welches Verbindungen des Rhodiums oder Iridiums, ein Jodid, ein tertiäres Phosphin und/oder ein tertiäres Phosphinoxid, z.B. Triphenylphosphinoxid, in einem inerten Lösemittel wie z.B. Toluol, Hexan, Octan, Cyclohexan etc. enthält. Weiterhin kann das Katalysatorsystem Phosphinate der Formel $(R)_2P(=O)OR$, Phosphorsäureester der Formel $(RO)_3PO$ oder Phosphorsäuretriamide der Formel $(R_2N)_3PO$ enthalten. Als Reaktionsprodukt fällt hierbei ein einphasiges, Katalysatorfeststoffanteile enthaltendes Gemisch an. Zur Isolierung des gewünschten Acetylhalogenids, z.B. des Acetylchlorids, sowie zur Rückgewinnung bzw. Kreislaufführung der Katalysatorbestandteile einschliesslich des Lösemittels und des nichtumgesetzten Alkylhalogenids, z.B. Methylchlorid, ist eine destillative Aufarbeitung des Reaktionsgemisches unumgänglich.

Dabei stellen sich durch weiteren Ausfall von Katalysatoranteilen, die ebenfalls im Kreislauf geführt werden müssen, erhebliche technische Schwierigkeiten ein. Durch Konzentrationsänderung während der destillativen Aufarbeitung kommt es weiterhin verstärkt zu Ablagerungen des salzartigen Katalysators in Rohrleitungen, Kolonnen und Verdampfern. Neben zwangsläufig auftretenden Behinderungen des Betriebsablaufes sind Verluste des teuren Katalysators nicht zu umgehen. Daneben führt die thermische Belastung des Katalysators in den Destillationsapparaten zu schnellem Aktivitätsverlust und erfordert damit ständigen Katalysatoraustausch.

Das Verfahren der Erfindung ermöglicht es, diese Nachteile auszuschliessen. Es wurde nämlich gefunden, dass der Zusatz von Verbindungen mindestens eines Metalles aus der 6. Nebengruppe des Periodensystems der Elemente in Verbindung mit Methyltrialkylphosphoniumjodid und/ oder Methyltriarylphosphoniumjodid und n-Heptan zu einem Katalysatorsystem aus Edelmetallverbindungen der 8. Nebengruppe des Periodensystems der Elemente, Jod(-verbindungen) und ggf. Trialkyl- und/oder Triarylphosphinoxid bei der Reaktion von Methylchlorid und CO unter ggf. erhöhtem Druck und bei erhöhter Temperatur eine Auftrennung des anfallenden Reaktionsproduktes in zwei Phasen bewirkt. Hierbei zeigt sich überraschenderweise, dass das gesamte Katalysatorsystem völlig gelöst die schwerere untere Phase bildet, während sich der überwiegende Teil des gebildeten Acetylchlorids in der oberen Lösemittelschicht befindet. Die Phasentrennung von Katalysatorsystem und Acetylchlorid/Lösemittel im Verfahren der Erfindung bietet gegenüber den bereits bekannten Herstellungsmethoden erhebliche Vorteile:

1. Der Katalysator wird durch Phasentrennung aus dem Reaktionsprodukt abgetrennt und gelangt zum Wiedereinsatz in den Reaktor. Die destillative, mit Ausfall fester Katalysatoranteile verbundene Rückgewinnung ist nicht mehr erforderlich.

2. Der Katalysator verbleibt im Reaktionssystem und gelangt nicht in die destillative Aufarbeitung, wodurch ein Ausfallen des Katalysators in den Destillationsapparaten und Katalysatorverluste vermieden werden.

3. Thermische Belastung des Katalysators und vorzeitiger Aktivitätsverlust durch die Aufarbeitung entfallen.

4. Neben der technisch besseren Durchführbarkeit des Verfahrens wird durch den Zusatz von Verbindungen des Cr, Mo oder W eine höhere Reaktionsgeschwindigkeit erzielt.

Die Edelmetalle Rhodium, Palladium, Iridium sowie die Metalle Chrom, Molybdän, Wolfram werden bevorzugt in Form ihrer Chloride (z.B. $RhCl_3 \cdot 3\,H_2O$, $CrCl_3 \cdot 6\,H_2O$), Acetate, Carbonyle oder als Komplexverbindungen, z.B. $Rh(CO)Cl[P(C_6H_5)_3]_2$, $Ir(CO)Cl[P(C_6H_5)_3]_2$, $RhCl[P(C_6H_5)_3]_3$, $[Rh(CO)_2Cl]_2$, $HIr(CO)[P(C_6H_5)_3]_3$, $HRh(CO)[P(C_6H_5)_3]_3$ eingesetzt.

Als Jodverbindung wird bevorzugt Methyljodid, aber auch Äthyljodid oder Jodwasserstoff eingesetzt.

Im Trialkylphosphinoxid und im Methyltrialkylphosphoniumjodid steht «alkyl» bevorzugt für methyl, äthyl, propyl, butyl. Im Triarylphosphinoxid und im Methyltriarylphosphoniumjodid steht «aryl» bevorzugt für phenyl.

Das Katalysatorsystem Edelmetall(-verbindung)/Verbindung des Cr, Mo oder W/Jod(-verbindung)/tert. Phospinoxid/quatern. Phosphoniumjodid/n-Heptan wird bevorzugt im Molverhältnis 1:(1–8):(1–100):(0–50):(1–100):(50–500) eingesetzt.

Je Mol Methylchlorid wird vorzugsweise 0,0001–0,01 Mol Edelmetall(-verbindung) und 0,0001–0,08 Mol der Verbindung(en) des Cr, Mo oder W eingesetzt.

Die Umsetzung gemäss der Erfindung erfolgt bevorzugt bei 20–180 bar und 150–250°C (423–523 K).

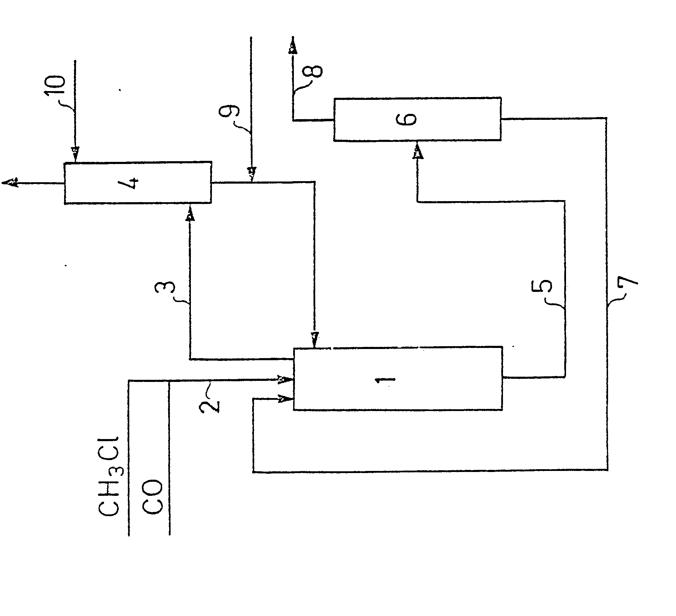

Zur Durchführung des Verfahrens der Erfindung ist in der Zeichnung ein mögliches Fliessschema dargestellt und im folgenden beschrieben.

In einem mit einer Mischeinrichtung versehenen Druckreaktor (1) werden Methylchlorid und Kohlenmonoxid aus Leitung (2) in Gegenwart eines Katalysatorsystems aus den Edelmetallen Rhodium, Palladium oder Iridium oder deren Verbindungen, Chrom, Molybdän oder Wolfram oder deren Verbindungen, Jod und/oder dessen Verbindungen, vorzugsweise Methyljodid, sowie Methyltrialkylphosphoniumjodid und/oder Methyltriarylphosphoniumjodid, vorzugsweise Methyltriphenylphosphoniumjodid, und ggf. Trialkylphosphinoxid oder Triarylphosphinoxid, vorzugsweise Triphenylphosphinoxid, in Anwesenheit von n-Heptan als inertem Lösemittel unter einem Druck von bevorzugt 20–180 bar und bei einer Temperatur von vorzugsweise 150° bis 250°C (423–523 K) zu Acetylchlorid umgesetzt. Eine geringe Menge gasförmiger Nebenprodukte wie Methan oder auch Verunreinigungen des eingesetzten Kohlenmonoxids wie Argon und Stickstoff werden mit nichtumgesetztem CO über Leitung (3) und eine Abgaswäsche (4) aus dem System entfernt. Umgesetztes Methylchlorid und CO werden über Leitung (2) durch Zusatz von Frischprodukten ergänzt. Das Reaktionsprodukt einschliesslich Katalysator wird über Leitung (5) dem Scheidegefäss (6) zugeführt. Hier trennt sich das Reaktionsprodukt in die Katalysator- (untere Phase) und Produktphase (obere Phase) auf. Entsprechend der dem Scheidegefäss zugeführten Menge zirkuliert die Katalysatorphase, die den gesamten Katalysator enthält, über Leitung (7) wieder zum Reaktionsgefäss (1), während das gebildete Acetylchlorid mit nichtumgesetztem Methylchlorid, Anteilen Methyljodid und n-Heptan über Leitung (8) abgezogen und in bekannter Weise durch Destillation getrennt wird. Nichtumgesetzte Ausgangsprodukte sowie das Lösemittel werden über die Leitungen (9) und (10) in die Anlage zurückgeführt.

Beispiel 1

1,95 g RhCl$_3$ · 3 H$_2$O, 7,91 g CrCl$_3$ · 6 H$_2$O, 3,4 g Triphenylphosphinoxid, 30 g Methyl-triphenylphosphoniumjodid, 15,8 g Methyljodid, 150,5 g Methylchlorid und 150,5 g n-Heptan werden in einen Autoklaven aus korrosionsbeständigem Edelstahl eingefüllt und unter einen CO-Druck von 75 bar gesetzt. Nach einer Reaktionszeit von 1 Stunde bei 453 K wird das Reaktionsprodukt dem Autoklaven entnommen. Im Scheidetrichter sind zwei feststofffreie Phasen zu erkennen. Nach getrenntem Aufdestillieren beider Phasen werden die gesamten salzartigen Bestandteile des eingesetzten Katalysators in der unteren Schicht wiedergefunden, während die obere Schicht praktisch rückstandsfrei destilliert. Es werden 110 g Acetylchlorid (47% d.Th.) ermittelt, woraus sich eine Raum-Zeit-Ausbeute von 275 g CH$_3$COCl/l Reaktionsvolumen und Stunde bzw. 145 g CH$_3$COCl/g Rh · h ergibt.

Beispiel 2

1,95 g RhCl$_3$ · 3 H$_2$O, 3,96 g CrCl$_3$ · 6 H$_2$O, 2,55 g Methyl-tri-n-butylphosphoniumjodid, 27,0 g Methyltriphenylphosphoniumjodid, 3,4 g Triphenylphosphinoxid, 15,8 g Methyljodid, 150,5 g Methylchlorid und 150,5 g n-Heptan werden in einen Autoklaven aus korrosionsbeständigem Edelstahl eingewogen und mit einem CO-Druck von 75 bar versehen. Nach einer Reaktionszeit von 1 Stunde bei 453 K fällt im Autoklaven ein zweiphasiges Reaktionsprodukt an. Die salzartigen Bestandteile des Katalysators befinden sich wiederum in der unteren Schicht, während die obere Schicht nahezu rückstandsfrei überdestilliert wird. An Acetylchlorid werden 119 g (51% d.Th.) gefunden, entsprechend einer Raum-Zeit-Ausbeute von 298 g CH$_3$COCl/l Rv · h bzw. 157 g CH$_3$COCl/g Rh · h.

Beispiel 3

1,95 g RhCl$_3$ · 3 H$_2$O, 15,8 g CrCl$_3$ · 6 H$_2$O, 3,4 g Triphenylphosphinoxid, 120 g Methyl-triphenylphosphoniumjodid, 15,8 g Methyljodid, 150,5 g Methylchlorid und 150,5 g n-Heptan werden in einen Autoklaven aus korrosionsbeständigem Edelstahl eingefüllt, mit einem CO-Druck von 75 bar versehen und bei einer Temperatur von 453 K umgesetzt. Nach einer Reaktionszeit von 0,8 Stunden wird aus dem Autoklaven ein zweiphasiges Reaktionsprodukt abgezogen, wobei auch diesmal die untere Schicht den Katalysator enthält. Es werden 122 g Acetylchlorid festgestellt (52% d.Th.), entsprechend einer Raum-Zeit-Ausbeute von 381 g CH$_3$COCl/l Rv · h bzw. 161 g CH$_3$COCl/g Rh · h.

Beispiel 4

0,98 g RhCl$_3$ · 3 H$_2$O, 3,96 g CrCl$_3$ · 6 H$_2$O, 30 g Methyl-triphenylphosphoniumjodid, 15,8 g Methyljodid, 150,5 g Methylchlorid und 150,5 g n-Heptan werden in einen Autoklaven aus korrosionsbeständigem Edelstahl eingewogen, mit einem CO-Druck von 75 bar versehen und eine Stunde bei 453 K zur Reaktion gebracht. Im Reaktionsprodukt werden 101 g Acetylchlorid (43% d.Th.) gefunden, entspechend einer Raum-Zeit-Ausbeute von 252 g CH$_3$COCl/l Rv · h bzw. 266 g CH$_3$COCl/g Rh · h.

Beispiel 5

1,95 g RhCl$_3$ · 3 H$_2$O, 7,91 g CrCl$_3$ · 6 H$_2$O, 3,4 g Triphenylphosphinoxid, 60 g Methyl-triphenylphosphoniumjodid, 15,8 g Methyljodid, 150,5 g Methylchlorid und 150,5 g n-Heptan werden in einen Autoklaven aus korrosionsbeständigem Edelstahl eingefüllt und unter einen CO-Druck von 75 bar gesetzt. Nach einer Reaktionszeit von 1 Stunde bei 453 K wird ein feststofffreies, zweiphasiges Reaktionsprodukt erhalten. Die Analyse ergibt 126 g Acetylchlorid (54% d.Th.), entsprechend einer Raum-Zeit-Ausbeute von 315 g $CH_3COCl/l$ Rv · h bzw. 166 g $CH_3COCl/g$ Rh · h.

Beispiel 6

1,95 g $RhCl_3$ · 3 $H_2O$, 7,91 g $CrCl_3$ · 6 $H_2O$, 3,4 g Triphenylphosphinoxid, 30 g Methyl-triphenylphosphoniumjodid, 15,8 g Methyljodid, 150,5 g Methylchlorid und 150,5 g n-Heptan werden in einen Autoklaven aus korrosionsbeständigem Edelstahl eingesetzt und mit einem CO-Druck von 75 bar versehen. Nach einer Reaktionszeit von 1 Stunde bei 453 K wird ein zweiphasiges, feststofffreies Reaktionsprodukt erhalten. Die untere Schicht wird in den Autoklaven zurückgegeben, wiederum mit den gleichen Mengen Methyljodid, n-Heptan und Methylchlorid versetzt und erneut unter einen CO-Druck von 75 bar gesetzt. Nach einer Reaktionszeit von 1 Stunde bei 453 K wird dem Autoklaven abermals ein zweiphasiges Reaktionsprodukt entnommen. Entsprechend dem erstmaligen Wiedereinsatz des Katalysators wird noch dreimal verfahren.

Nach insgesamt 4-maligem Zurückleiten des Katalysators wird der Versuch beendet. Bei einer Gesamtreaktionszeit von 5 Stunden werden 505 g Acetylchlorid (43% d.Th.) erhalten. Die Raum-Zeit-Ausbeute erreicht einen Wert von 253 g $CH_3COCl/l$ Rv · h, die Katalysatorleistung beträgt 133 g $CH_3COCl/g$ Rh · h.

Beispiel 7

1,95 g $RhCl_3$ · 3 $H_2O$, 7,91 g $CrCl_3$ · 6 $H_2O$, 3,4 g Triphenylphosphinoxid, 120 g Methyl-triphenylphosphoniumjodid, 15,8 g Methyljodid, 150,5 g Methylchlorid und 150,5 g n-Heptan werden in einen Autoklaven aus korrosionsbeständigem Edelstahl eingefüllt und unter einem CO-Druck von 75 bar bei 453 K umgesetzt. Nach einer Reaktionszeit von 0,6 Stunden wird das Zweiphasengemisch dem Autoklaven entnommen. Auch hierbei wird der Katalysator in der unteren Schicht wiedergefunden. An Acetylchlorid werden 115 g (49% d.Th.) ermittelt, woraus sich eine Raum-Zeit-Ausbeute von 479 g $CH_3COCl/l$ Rv · h bzw. 252 g $CH_3COCl/g$ Rh · h errechnet.

Beispiel 8 (Vergleichsbeispiel)

1,95 g $RhCl_3$ · 3 $H_2O$, 3,4 g Triphenylphosphinoxid, 77,9 g Triphenylphosphin, 15,8 g Methyljodid, 150,5 g Methylchlorid und 150 g n-Octan werden in einen Autoklaven aus korrosionsbeständigem Edelstahl eingefüllt und mit einem CO-Druck von 75 bar vesetzt. Nach einer Reaktionszeit von 1 Stunde bei 453 K wird das Reaktionsprodukt dem Autoklaven entnommen. Hierbei zeigt sich, dass ein grosser Teil des eingesetzten Katalysatorgemisches in Form von schmierigen bis kristallinen Ablagerungen im Reaktionsgemisch vorliegt. Nach Abtrennen vom festen Rückstand, Aufdestillieren der verbleibenden flüssigen Phase und Analysieren werden 39 g Acetylchlorid (16,7% d.Th.) gefunden, entsprechend einer Raum-Zeit-Ausbeute von 98 g $CH_3COCl/l$ Rv · h bzw. 51 g $CH_3COCl/g$ Rh · h.

Beispiel 9

Ein aus Edelstahl bestehender Autoklav ist mit 1,5 l Reaktionsgemisch gefüllt. Das Reaktionsgemisch enthält 18 mMol Rh/l, die als $RhCl_3$ · 3 $H_2O$ eingesetzt worden waren. Es liegt ein molares Verhältnis von Rhodiumverbindung : Chromverbindung : Methyljodid : Triphenylphosphinoxid : Methyltriphenylphosphoniumjodid : n-Heptan = 1:4:15:1,65:20:202 vor. Dem Reaktionsgefäss werden stündlich 264 g Frischmethylchlorid zugeführt. Der Reaktionsdruck wird durch kontinuierliche Zufuhr von CO auf 75 bar gehalten, die Reaktionstemperatur beträgt 450 bis 455 K. Das entsprechend der Zufuhr abfliessende Reaktionsprodukt wird einem Scheidegefäss zugeführt, in dem es sich in zwei Phasen trennt. Die untere Schicht, die dem Reaktor wieder zugeleitet wird, enthält die Katalysatorlösung. Die aus dem Scheidegefäss abfliessende obere Produktphase enthält neben n-Heptan etwa 37 Gew.% Acetylchlorid, etwa 6 Gew.% Methylchlorid und etwa 10 Gew.% Methyljodid und wird in an sich bekannter Weise aufgearbeitet. Dabei fallen stündlich 390 g Acetylchlorid an. Nichtumgesetztes Methylchlorid, Methyljodid und n-Heptan werden in den Reaktor zurückgegeben. Bei einer Ausbeute an Acetylchlorid, bezogen auf zugeführtes Methylchlorid, von 95% ergibt sich eine Raum-Zeit-Ausbeute von 260 g $CH_3COCl/l$ Rv · h bzw. eine Katalysatorleistung von 140 g $CH_3COCl/g$ Rh · h.

**Patentansprüche**

1. Verfahren zur Herstellung von Acetylchlorid durch Umsetzung von Methylchlorid mit Kohlenmonoxid unter praktisch wasserfreien Bedingungen bei Temperaturen von 350 bis 575 K und Drücken von 1 bis 300 bar in Gegenwart eines Katalysatorsystems, welches mindestens eines der Edelmetalle Rhodium, Palladium, Iridium oder deren Verbindungen, Jod und/oder dessen Verbindungen, ggf. Trialkylphosphinoxid oder Triarylphosphinoxid, sowie ein inertes organisches Lösemittel enthält, dadurch gekennzeichnet, dass man in Gegenwart eines Katalysatorsystems arbeitet, welches n-Heptan als inertes Lösemittel und zusätzlich Methyltrialkylphosphoniumjodid und/oder Methyltriarylphosphoniumjodid und mindestens eine Verbindung des Chroms, Molybdäns oder Wolframs enthält.

2. Verfahren nach Anspruch 1, dadurch gekennzeichnet, dass man das Katalysatorsystem Edelmetall(-verbindung)/Verbindung des Cr, Mo oder W/Jod(-verbindung)/tert. Phosphinoxid/quatern. Phosphoniumjodid/n-Heptan im Molverhältnis 1:(1–8):(1–100):(0–50):(1–100):(50–500) einsetzt.

**Claims**

1. Process for making acetyl chloride by reacting methyl chloride with carbon monoxide under practically anhydrous conditions, at temperatures of 350 to 575 K, under pressures of 1 to 300 bars, in the presence of a catalyst system containing at least one of the noble metals rhodium, palladium, iridium or their compounds, iodine and/or its compounds, optionally trialkylphosphine oxide or triarylphosphine oxide, as well as an inert organic solvent, charcterised in that the reaction is effected in the presence of a catalyst system containing n-heptane as inert solvent and additionally methyltrialkylphosphonium iodide and/or methyltriarylphosphonium iodide and at least one compound of chromium, molybdenum or tungsten.

2. Process as claimed in claim 1, characterised in that the catalyst system noble metal (-compound)/compound of Cr, Mo or W/iodine(-compound)/tertiary phosphine oxide/quaternary phosphonium iodide/n-heptane is used in a molar ratio of 1:(1–8):(1–100):(0–50):(1–100):(50–500).

**Revendications**

1. Procédé de préparation du chlorure d'acétyle par réaction du chlorure de méthyle avec le monoxyde de carbone en conditions pratiquement anhydres à des températures de 350 à 575 K et sous des pressions de 1 à 300 bars en présence d'un système catalytique contenant au moins l'un des métaux nobles rhodium, palladium, iridium ou leurs composés, de l'iode et/ou ses composés, éventuellement un oxyde de trialkylphosphine ou de triarylphosphine ainsi qu'un solvant organique inerte, caractérisé en ce que l'on opère en présence d'un système catalytique contenant du n-heptane comme solvant inerte et, comme composants supplémentaires, un iodure de méthyltrialkylphosphonium et/ou de méthyltriarylphosphonium et au moins un composé de chrome, de molybdène ou de tungstène.

2. Procédé selon la revendication 1, caractérisé en ce que l'on utilise un système catalytique contenant le (composé de) métal noble/composé de Cr, Mo ou W/(composé d')iode/oxyde de phosphine tertiaire/iodure de phosphonium quaternaire/n-heptane dans le rapport molaire de 1:(1–8):(1–100):(0–50):(1–100):(50–500).